# FASCICULE DE BREVET EUROPEEN

(11) **EP 4 332 572 B1**
(45) Date de publication et mention de la délivrance du brevet: **14.01.2026**
(21) Numéro de dépôt: 23193750.9
(22) Date de dépôt: 28.08.2023
(51) Int. Cl.: B01L 3/00, G01N 33/49

(54) **DISPOSITIF INSTRUMENTÉ EMPLOYÉ POUR LA DÉTECTION DE PRÉSENCE DE MICRO-ORGANISMES DANS UN ÉCHANTILLON LIQUIDE**
INSTRUMENTIERTE VORRICHTUNG ZUR ERKENNUNG DES VORHANDENSEINS VON MIKROORGANISMEN IN EINER FLÜSSIGEN PROBE
INSTRUMENTED DEVICE USED FOR DETECTING THE PRESENCE OF MICROORGANISMS IN A LIQUID SAMPLE

(30) Priorité: 01.09.2022 FR 2208762
(43) Date de publication de la demande: 06.03.2024
(73) Titulaire: Commissariat à l'Energie Atomique et aux Energies Alternatives, 75015 Paris (FR)
(72) Inventeur: BABIN, Thibaut, 38054 Grenoble cedex 09 (FR); MARCOUX, Pierre, 38054 Grenoble cedex 09 (FR); MAILLEY, Pascal, 38054 Grenoble cedex 09 (FR); GOUGIS, Maxime, 38054 Grenoble cedex 09 (FR); RAFIE JIRDEHI, Mehrsa, 38054 Grenoble cedex 09 (FR)
(74) Mandataire: INNOV-GROUP

(56) Documents cités:
- WO-A1-2014/087137
- CN-A- 105 092 668
- CN-A- 112 748 168
- JP-A- H08 173 391
- US-A- 4 322 279

## Description

### Domaine technique de l'invention

La présente invention se rapporte à un dispositif instrumenté adaptable sur un conteneur contenant un échantillon liquide, ledit dispositif pouvant être employé pour la détection de présence de micro-organismes dans ledit échantillon.

### Etat de la technique

Le sang est en temps normal totalement stérile. Tout micro-organisme présent dans le système sanguin représente donc une menace vitale pour le corps humain. L'hémoculture est actuellement une méthode connue de diagnostic des infections sanguines. Il s'agit d'un test fréquent en microbiologie clinique. L'hémoculture consiste en premier lieu en la culture du sang dans un bouillon nutritif en condition aérobie et anaérobie, sous incubation à 36-37°C dans des automates adaptés à ce type d'échantillons, afin d'obtenir une croissance bactérienne. L'objectif consiste à amplifier la quantité de bactéries présentes dans l'échantillon, en lui fournissant des conditions favorables de croissance, et ainsi à détecter la présence de bactérie dans le sang chez un patient présentant un état septique. Cette amplification est d'autant plus nécessaire que la concentration bactérienne dans le sang au cours des bactériémies est toujours faible, de l'ordre d'une ou de quelques bactéries par millilitre de sang prélevé. Cette première étape de l'hémoculture consiste donc en une simple détection de présence, sans identification, à partir d'un échantillon sans flore normale : toute présence de bactérie ou champignon conduit donc à un test positif.

Cette détection sera ensuite suivie d'une subculture sur milieu gélosé pour isoler le pathogène sous forme de colonies, permettant ensuite de procéder à l'identification du pathogène et à son antibiogramme. Dans le cas des infections sanguines, les examens sont urgents et le délai de rendu des résultats (positivité de l'hémoculture, identité du pathogène, antibiogramme) a un impact indéniable sur le devenir des patients (mortalité, durée d'hospitalisation, complications ...). Les résultats d'hémoculture permettent en effet d'adapter l'antibiothérapie au cas spécifique du patient : plus une antibiothérapie adaptée et efficace intervient précocement, plus les chances de survie du patient s'accroissent. Chaque heure de retard à l'instauration d'une antibiothérapie adaptée est associée à une augmentation de la mortalité.

Les industriels du diagnostic ont œuvré pendant de nombreuses années à réduire le délai de positivité des hémocultures, ainsi qu'à réduire les temps d'analyse sur les tests d'identification et d'antibiogramme. Actuellement, deux grands types d'automates coexistent :
- Des appareils qui suivent la quantité d'acide carbonique généré dans la phase liquide, grâce à une matrice polymère (silicone) chargée avec un chromophore ou un fluorophore sensible au pH.
- Des appareils employés pour déceler une augmentation de la pression totale dans la phase gaz.

Ces deux technologies ont en commun d'être mises en œuvre dans des automates, souvent de grande taille, avec l'impossibilité de démarrer le test tant que le flacon n'est pas dans l'automate (une pré-incubation peut générer des faux-négatifs), d'où la perte de précieuses heures avant la mise en place d'une antibiothérapie optimisée. Une étude en 2013 a montré que le temps de transport moyen était de 9 h (écart interquartile : 3-15 h), avec 6% de flacons présentant un temps de transport supérieur à 20 h.

La demande de brevet EP4018191A1 décrit l'utilisation d'un conteneur instrumenté doté d'électrodes de mesure, pouvant accueillir un échantillon liquide à analyser. La détection de présence de micro-organismes est notamment réalisable par électrochimie, en plaçant le conteneur dans un caisson adapté. Cette demande de brevet propose une solution simple permettant de réduire la durée entre le prélèvement de l'échantillon et le démarrage de l'analyse de l'échantillon prélevé. Cette solution est par ailleurs facilement transportable, la rendant disponible sur le terrain, et facile à mettre en œuvre, même par du personnel peu qualifié.

Cependant, cette solution oblige à développer des conteneurs particuliers et non standards, ce qui représente un coût non négligeable.

La demande de brevet WO2014/087137A1 décrit une solution capable de réaliser des mesures rapides sur des échantillons biologiques. Le brevet US4322279 décrit également un dispositif d'analyse d'échantillons.

Le but de l'invention est de proposer une solution pour détecter la présence d'un micro-organisme dans un échantillon liquide, qui soit rapidement déployable après le prélèvement, pour conserver une durée réduite entre le prélèvement de l'échantillon et le démarrage de l'analyse de l'échantillon prélevé, tout en étant adaptable aux flacons de prélèvement existants. En effet, ces flacons sont préremplis avec un milieu de culture dont la composition est optimisée pour la réalisation de tests de stérilité.

La solution de l'invention permet notamment d'éviter toute dégradation des électrodes lors d'une analyse.

### Exposé de l'invention

Ce but est atteint par un dispositif instrumenté adaptable sur un conteneur comprenant une enveloppe destinée à recevoir un échantillon liquide, le dispositif comprenant au moins deux électrodes, dites électrode de mesure et électrode de référence :
- Le dispositif instrumenté comportant au moins un élément de mesure (2) réalisé en matériau isolant de l'électricité,
- Ledit élément de mesure comportant des moyens pour coopérer de manière amovible avec l'enveloppe dudit conteneur,
- Ledit élément de mesure comportant une première surface destinée à venir en contact avec un volume interne dudit conteneur lorsque ledit élément de mesure est adapté sur le conteneur, et une deuxième surface opposée accessible de l'extérieur,
- La première surface dudit élément de mesure portant ladite électrode de mesure et ladite électrode de référence,
- La deuxième surface dudit élément de mesure portant un premier organe conducteur de l'électricité et un deuxième organe conducteur de l'électricité,
- Ledit élément de mesure intégrant une première traversée électrique reliant ladite électrode de mesure au premier organe conducteur et une deuxième traversée électrique reliant ladite électrode de référence au deuxième organe conducteur,
- L'élément de mesure comportant une tige allongée suivant un axe dit longitudinal réalisée en matériau isolant de l'électricité, la tige comprenant une extrémité proximale et une extrémité distale, ladite tige portant à son extrémité proximale ledit premier organe conducteur de l'électricité et ledit deuxième organe conducteur de l'électricité, la tige comprenant une surface externe destinée à venir en contact avec l'échantillon liquide, sur laquelle sont réalisées ladite électrode de mesure et ladite électrode de référence,
- La tige comportant une première cavité réalisée sur sa surface externe, ladite électrode de référence étant déposée dans ladite première cavité,
- La tige comportant une deuxième cavité réalisée sur sa surface externe, ladite électrode de mesure étant déposée dans ladite deuxième cavité.

Selon une réalisation particulière, l'élément de mesure comporte un bouchon réalisé en matériau isolant de l'électricité et comprenant des moyens de coopération par vissage ou emboîtement, destinés à coopérer avec des moyens complémentaires agencés sur un goulot du conteneur.

Selon une autre particularité, la tige comporte une pointe à son extrémité distale. Selon une autre particularité, la tige est creuse sur toute sa longueur.

Selon une autre particularité, l'élément de mesure comporte une tête positionnée à l'extrémité proximale de la tige, la tête portant ledit premier organe conducteur et ledit deuxième organe conducteur.

Selon une autre particularité, le dispositif comporte des moyens d'assemblage de la tête sur la tige.

Selon une autre particularité, la tige et la tête coopèrent entre elles par vissage.

Selon une autre particularité, la tête comporte des moyens de coopération par vissage ou emboîtement, destinés à coopérer avec des moyens complémentaires agencés sur un goulot du conteneur.

Selon une variante particulière, la tige est réalisée à partir d'une carte électronique réalisée en matériau souple et enroulée sur elle-même.

Selon une particularité, ladite électrode de mesure est réalisée sous la forme d'un dépôt d'une encre conductrice ou d'un électro-dépôt d'un élément de mesure conducteur. Selon une autre particularité, ladite électrode de référence est réalisée sous forme d'un dépôt d'une encre Ag/AgCl recouverte d'une couche polymère.

### Brève description des figures

D'autres caractéristiques et avantages vont apparaître dans la description détaillée qui suit faite en regard des dessins annexés dans lesquels :
- Les figures 1A à 1C montrent un premier mode de réalisation du dispositif instrumenté ne faisant pas partie de l'invention ;
- Les figures 2A et 2B montrent un deuxième mode de réalisation du dispositif instrumenté conforme à l'invention ;
- Les figures 3A et 3B représentent un troisième mode de réalisation du dispositif instrumenté conforme à l'invention ;
- Les figures 4 à 6 illustrent différentes variantes de réalisation du dispositif instrumenté, applicables au deuxième mode de réalisation et au troisième mode de réalisation ;

### Description détaillée d'au moins un mode de réalisation

L'invention vise un dispositif instrumenté employé pour la détection de présence de micro-organismes dans un échantillon liquide ECH, placé dans un conteneur 1.

L'échantillon liquide ECH peut être un fluide biologique, avantageusement choisi dans le groupe constitué par le sang tel que du sang entier ou du sang entier anti-coagulé, le sérum sanguin, le plasma sanguin, la lymphe, les larmes, le sperme, l'urine, le lait, le liquide céphalo-rachidien, le liquide interstitiel, un liquide articulaire, un liquide péricardique, un fluide isolé de moelle osseuse, un extrait cellulaire, un extrait tissulaire, un extrait d'organes et un de leurs mélanges. Ainsi, le fluide biologique peut être tout fluide naturellement sécrété ou excrété d'un corps humain ou animal ou tout fluide récupéré, à partir d'un corps humain ou animal, par toute technique connue de l'homme du métier telle qu'une extraction, un prélèvement, une ponction ou un lavage. Les étapes de récupération et d'isolement de ces différents fluides à partir du corps humain ou animal sont réalisées préalablement et ne font pas partie de l'invention.

L'échantillon liquide ECH peut également être un produit liquide issu d'une industrie agro-alimentaire, pharmaceutique ou cosmétique. Dans un mode de réalisation particulier, l'objet sur lequel le prélèvement est effectué peut être choisi parmi des installations de grande taille comme un objet industriel comme un appareil électronique ou une machine utilisée dans les industries agro-alimentaires, pharmaceutiques ou cosmétiques, une cuve, une cuisine de restaurant, une chambre froide, un sanitaire, un conteneur, et des objets de petite taille comme des dispositifs médicaux ou des tuyaux. L'échantillon liquide ECH peut également être un milieu de culture stérile, entourant un échantillon solide ou pulvérulent dont on veut tester la stérilité, par exemple une biopsie, des tissus pour greffes, un dispositif médical (valve cardiaque, prothèse, lentille de contact, seringue, aiguille, etc.), un principe actif en poudre, du matériel de laboratoire à usage unique, un équipement chirurgical...

Dans un mode de réalisation plus particulier, l'échantillon liquide est du sang comme du sang humain ou animal. Ce dernier est normalement stérile mais peut contenir des micro-organismes comme des bactéries.

En référence aux figures annexées, le dispositif comporte un élément de mesure portant au moins deux électrodes E1, E2 différentes, utilisées pour analyser l'échantillon liquide ECH, une électrode de mesure E1 et une électrode de référence E2.

L'électrode de mesure E1 utilisée dans le dispositif de l'invention est avantageusement une électrode réalisée sous forme d'un dépôt d'une encre conductrice ou une électrode réalisée sous forme d'un électro-dépôt d'un élément de mesure conducteur. Toute encre conductrice connue de l'homme du métier peut être mise en œuvre. Dans un mode de réalisation particulier, l'encre conductrice utilisée est une encre à base d'ions métalliques ou de polymères organiques conducteurs tels que le polythiophène (PT), la polyaniline (PANI) éventuellement dopée avec de l'acide dodecylbenzènesulfonique (DBSA), le poly(3,4-éthylènedioxythiophène) couplé au poly(styrène sulfonate) de sodium (PEDOT:PSS), le polypyrrole ou le polyphthalocyanine. Dans un autre mode de réalisation particulier, l'encre conductrice utilisée dans le cadre de l'invention est une encre carbone comprenant éventuellement un élément de mesure conducteur additionnel. Par "élément de mesure conducteur", on entend un élément de mesure choisi dans le groupe constitué par les polymères organiques conducteurs tels que ceux listés ci-dessus, et les composés à base de métal comme, par exemple, un oxyde métallique tel que l'oxyde d'iridium (IrOx), un pigment à base de métal tel que le bleu de Prusse (Fe(III) 5 ferrocyanure) ou encore un catalyseur organique ou organo-inorganique tel que de la phtalocyanine de cobalt.

Avantageusement, l'électrode de référence E2 utilisée dans le cadre de l'invention est réalisée sous forme d'un dépôt d'une encre Ag/AgCl.

De manière optionnelle, Il est possible de mettre une contre-électrode en contact avec le milieu de culture liquide.

Le dispositif de l'invention est intégré dans un système configuré pour détecter et éventuellement identifier un micro-organisme contenu dans l'échantillon liquide ECH placé dans le conteneur 1.

Le système comporte également :
- Une unité de mesure U1 de la différence de potentiel entre l'électrode de mesure E1 et l'électrode de référence E2 et configurée pour mesurer la différence de potentiel, en continu et/ou à une pluralité d'instants temporels,
- Une unité électronique d'acquisition U2 de la mesure de la différence de potentiel, reliée à l'unité de mesure,
- Une unité de traitement U3 reliée à l'unité électronique d'acquisition U2 et configurée pour traiter la mesure de la différence de potentiel,
- Une unité d'alimentation électrique U4 pour alimenter l'unité électronique d'acquisition U2 et éventuellement l'unité de traitement. U3

Une unité de chauffage peut également être ajoutée, ainsi qu'une unité de mesure et de gestion de la température. Le chauffage devra être réalisé de manière homogène, tout autour du conteneur. L'unité de mesure et de gestion de la température peut comporter une sonde de température et des moyens de régulation de ladite température à une valeur fixe déterminée. La valeur de température est choisie en fonction du type de culture mise en œuvre. Dans le cas d'une hémoculture, la température d'incubation est choisie égale à 35°C +/- 2°C.

L'unité d'alimentation électrique U4 peut être constituée d'une batterie rechargeable destinée à alimenter les différentes unités du dispositif.

L'unité de traitement U3 peut comporter un microprocesseur et des moyens de mémorisation. Elle peut également comporter un module de communication. Ce module peut être sans-fil pour communiquer via une liaison sans-fil, par exemple selon le protocole Bluetooth, avec le module correspondant de l'unité d'acquisition.

A titre d'exemple, l'unité de traitement U3 peut être configurée pour :
- Recevoir les données de mesure en provenance de l'unité d'acquisition, par exemple via la liaison sans-fil,
- Etablir un suivi électrochimique du mélange en fonction du temps,
- Traiter la courbe obtenue et déterminer la présence de micro-organismes,
- Commander une interface homme-machine destinée à indiquer la présence de bactéries,
- Lire une étiquette RFID présente sur le conteneur 1 en vue de récolter et de stocker les données liées au prélèvement,
- Assurer la régulation de l'unité de chauffage pour assurer une température d'incubation la plus constante possible.

Le dispositif de l'invention présente la particularité de venir coopérer de manière amovible avec le conteneur 1 dans lequel est placé l'échantillon liquide ECH à analyser. Il se présente sous la forme d'un élément de mesure 2 doté de moyens adaptés pour coopérer avec le conteneur 1.

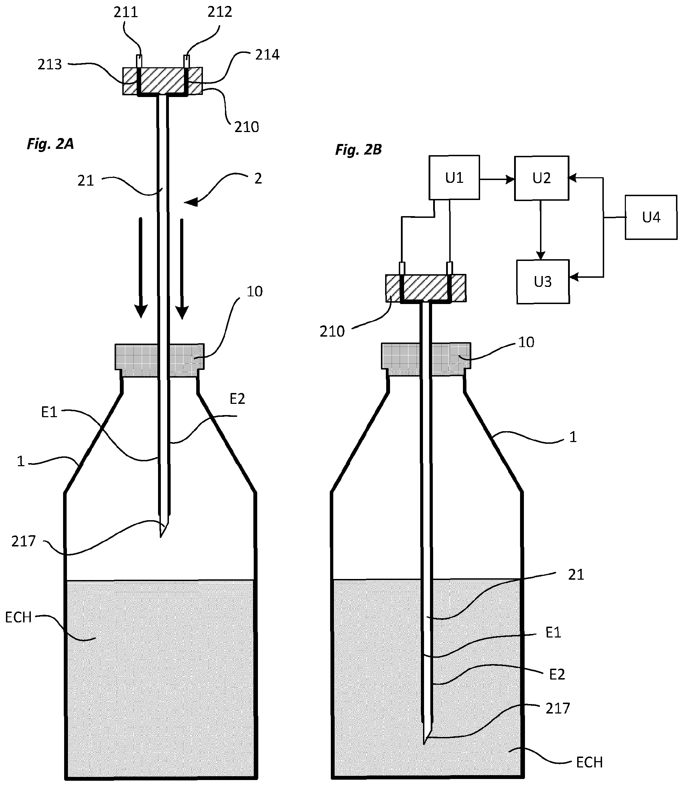

Le conteneur 1 peut être un conteneur standard, couramment utilisé pour stocker un prélèvement tel qu'un prélèvement sanguin. Un tel conteneur 1 peut notamment être fermé de manière hermétique à l'aide d'un septum (voir mode de réalisation des figures 2A et 2B). Le conteneur 1 peut également être tout récipient dont le contenu liquide (sérum physiologique, médicament injectable, milieu nutritif) est amené à se prêter à un test de stérilité.

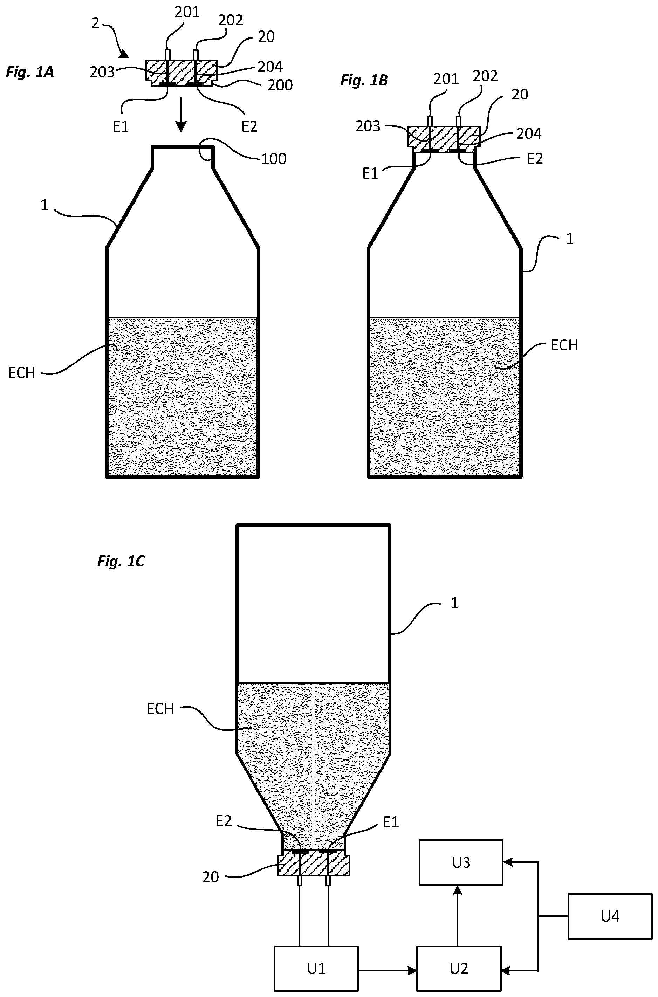

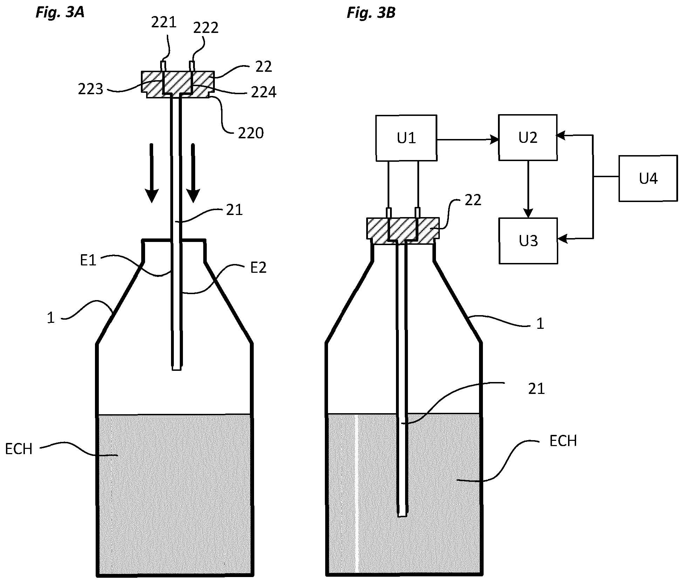

On distingue plusieurs modes de réalisation distincts du dispositif :
- Dans un premier mode de réalisation ne faisant pas partie de l'invention, représenté sur les figures 1A à 1C, l'élément de mesure 2 comporte un bouchon 20 venant se visser ou s'emboîter sur le conteneur 1, ce bouchon portant directement les deux électrodes E1, E2 ;
- Dans un deuxième mode de réalisation représenté sur les figures 2A et 2B, l'élément de mesure 2 comporte une tige 21 portant les deux électrodes E1, E2, la tige étant destinée à être insérée à l'intérieur du conteneur 1, plongée au moins partiellement dans l'échantillon liquide ECH, par exemple en traversant le septum 10 qui ferme le conteneur 1 de manière hermétique ;
- Dans un troisième mode de réalisation représenté sur les figures 3A et 3B, l'élément de mesure 2 se présente sous la forme d'un ensemble monobloc composé d'un bouchon 22 et d'une tige 21, assemblée sur ledit bouchon 22 et portant les deux électrodes E1, E2, le bouchon 22 étant doté de moyens pour venir s'adapter sur le conteneur, la tige 21 insérée dans le volume interne du conteneur 1 pour être plongée au moins partiellement dans l'échantillon liquide ECH ;

Le premier mode de réalisation, hors invention, est illustré par les figures 1A à 1C. Le bouchon 20 est réalisé dans un matériau isolant de l'électricité, par exemple en caoutchouc ou plastique. Il comporte des moyens de fixation 201, par vissage ou emboîtement, destinés à coopérer avec des moyens 100 complémentaires présents sur le conteneur 1, par exemple au niveau de son goulot. Il comporte une surface dite externe, accessible de l'extérieur et une surface interne, destinée à être orientée vers l'intérieur du conteneur lorsqu'il est adapté sur le conteneur.

Le bouchon 20 porte sur sa surface externe deux organes conducteurs 201, 202 de l'électricité et sur sa surface interne les deux électrodes, l'électrode de mesure E1 et l'électrode de référence E2, avantageusement réalisées chacune sous la forme d'un dépôt d'une couche de matière, selon l'une des réalisations proposées ci-dessus.

Entre le premier organe conducteur et l'électrode de mesure, le bouchon 20 intègre une première traversée électrique 203 permettant d'assurer la liaison électrique. Entre le deuxième organe conducteur et l'électrode de référence, le bouchon intègre une deuxième traversée électrique 204 permettant d'assurer la liaison électrique.

L'unité de mesure décrite ci-dessus est connectée entre les deux électrodes, pour mesurer la différence de potentiel entre les deux électrodes.

En fonctionnement, le bouchon 20 est positionné sur le goulot du conteneur 1 (figure 1A). Une fois le bouchon en place sur le conteneur (figure 1B), le conteneur peut être retourné pour mettre en contact l'échantillon liquide ECH avec les deux électrodes, le temps de la mesure (figure 1C).

Le bouchon 20 est bien entendu adapté sur le conteneur 1 pour le fermer de manière totalement hermétique, malgré la surpression inhérente à la croissance microbienne qui peut éventuellement se produire.

Dans le deuxième mode de réalisation représenté sur les figures 2A et 2B, la tige 21 est réalisée dans un matériau isolant de l'électricité. Elle comporte une extrémité proximale et une extrémité distale. La tige 21 comporte une surface externe, notamment une surface latérale sur laquelle sont réalisées les deux électrodes E1, E2. Chaque électrode est par exemple déposée sous la forme d'une couche de matière sur cette surface latérale de la tige 21.

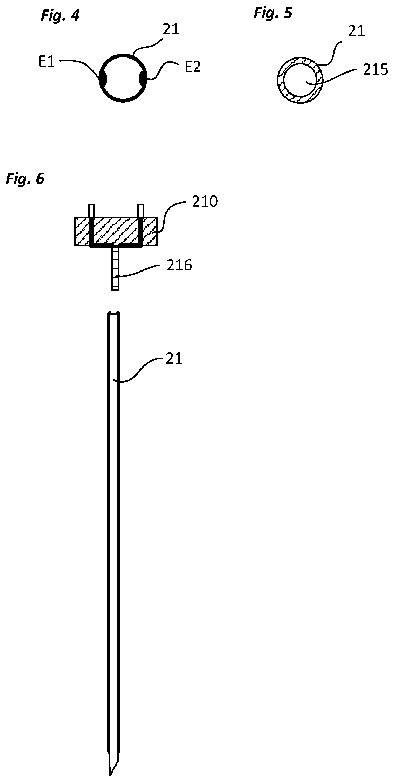

Selon l'invention, la paroi latérale de la tige est creusée, par exemple de deux cavités, par exemple sous la forme de tranchées longitudinales (figure 4). Chaque cavité ou tranchée reçoit un dépôt de matière formant respectivement l'électrode de mesure E1 et l'électrode de référence E2. La cavité ou la tranchée permet de mettre la surface de contact de l'électrode en retrait par rapport à la surface externe de la tige 21, empêchant sa dégradation notamment lors de l'insertion de la tige à travers le septum.

La tige 21 peut être configurée de manière à disposer d'une pointe 217 à son extrémité distale (comme sur les figures 2A et 2B), de manière à pouvoir percer le septum 10 fermant le conteneur de manière hermétique.

Une reprise de contact est réalisée du côté de l'extrémité proximale de la tige pour venir connecter chaque électrode E1, E2 à l'unité de mesure U1.

Dans cette variante, de manière avantageuse, l'élément de mesure 2 peut également comporter une tête 210 assemblée sur la tige 21, du côté de son extrémité proximale. La tête 210 est réalisée dans un matériau isolant de l'électricité. La tête 210 est accessible de l'extérieur lorsque l'élément de mesure 2 est adapté sur le conteneur. La tête 210 porte ainsi un premier organe conducteur 211 et un deuxième organe conducteur 212. Le premier organe conducteur est relié à l'électrode de mesure E1 et le deuxième organe conducteur est relié à l'électrode de référence E2, chacune via une traversée électrique 213, 214 intégrée dans la tête 210 de l'élément de mesure 2.

En fonctionnement, la tige 21 est insérée dans le conteneur 1 contenant l'échantillon liquide ECH, son extrémité distale en pointe venant percer le septum 10 (figure 2A). La tige est enfoncée dans le conteneur jusqu'à plonger les deux électrodes E1, E2 dans l'échantillon liquide ECH pour faire les mesures (figure 2B). La tête 210 reste accessible de l'extérieur pour venir connecter l'unité de mesure U1.

Le tige 21 peut être de section transversale pleine. Mais comme illustré par la figure 5, la tige 21 peut être réalisée de manière à être creuse, c'est-à-dire avec un canal interne 215 sur toute sa longueur, le long de son axe longitudinal. Cette particularité présente l'avantage de pouvoir utiliser également le dispositif pour le prélèvement de l'échantillon liquide ECH, pendant ou après la mesure électrochimique réalisée entre les deux électrodes E1, E2.

En référence à la figure 6, la tête 210 peut être assemblée sur la tige 21 par vissage (avec un pas de vis 216).

Le troisième mode de réalisation est une combinaison des deux premiers modes, en ce que l'élément de mesure 2 est doté de la tige 21 portant les deux électrodes E1, E2, identique à celle décrite ci-dessus, et d'une tête réalisée sous la forme d'un bouchon 22 sur lequel est fixée la tige 21. Le bouchon 22 comporte des moyens de fixation 220 par vissage ou emboîtement, permettant à l'élément de mesure 2 de venir s'adapter directement sur le goulot du conteneur 1 pour le fermer de manière hermétique, la tige 21 étant insérée vers l'intérieur du conteneur 1 pour être plongée dans l'échantillon liquide. Les autres particularités décrites ci-dessus pour le deuxième mode de réalisation sont identiques. Le bouchon 22 porte deux organes de contact électrique 221, 222 et intègrent deux traversées électriques 223, 224 pour les relier aux deux électrodes E1, E2. Dans ce mode de réalisation :
- Le bouchon 22 peut être assemblée sur la tige 21, de manière amovible, par exemple par vissage ;
- La configuration en pointe de la tige à son extrémité distale est optionnelle, de même que la présence du canal interne ;

Sur la figure 3A, le bouchon 22 portant la tige 21 est inséré dans le conteneur 1, jusqu'à venir s'adapter sur le goulot du conteneur 1. Sur la figure 3B, le bouchon est positionné de manière à fermer le conteneur 1 de manière hermétique, la tige plongée dans l'échantillon liquide ECH de manière à mettre en contact les électrodes E1, E2 avec l'échantillon liquide ECH. L'unité de mesure U1 peut ensuite être commandée pour réaliser les mesures.

Partant de ces différents modes de réalisation, il faut noter que :
- Il est possible de prévoir plus de deux électrodes sur l'élément de mesure. Le nombre de connexions électriques devra être adapté - on dispose alors d'un suivi électrochimique multiparamétrique ;
- La présence des tranchées, présente l'avantage d'éviter toute détérioration des dépôts lorsque la tige est insérée dans le conteneur à travers le septum ;
- Chaque cavité/tranchée réalisée sur la tige peut accueillir plusieurs électrodes de manière juxtaposée sur sa longueur ; Il est également possible de réaliser plusieurs cavités distinctes, une pour chaque électrode ;
- La tige peut être réalisée sous la forme d'une carte électronique souple sur une face de laquelle sont déposées les électrodes, la carte électronique étant enroulée sur elle-même pour donner sa forme allongée à ladite tige ;

## Revendications

1. Dispositif instrumenté adaptable sur un conteneur (1) comprenant une enveloppe destinée à recevoir un échantillon liquide (ECH), le dispositif comprenant :
- Au moins deux électrodes, dites électrode de mesure (E1) et électrode de référence (E2), :
- Au moins un élément de mesure (2) réalisé en matériau isolant de l'électricité,
- Ledit élément de mesure (2) comportant des moyens pour coopérer de manière amovible avec l'enveloppe dudit conteneur,
- Ledit élément de mesure (2) comportant une première surface destinée à venir en contact avec un volume interne dudit conteneur lorsque ledit élément de mesure (2) est adapté sur le conteneur (1), et une deuxième surface opposée accessible de l'extérieur,
- La première surface dudit élément de mesure (2) portant ladite électrode de mesure (E1) et ladite électrode de référence (E2),
- La deuxième surface dudit élément de mesure (2) portant un premier organe conducteur (201, 211, 221) de l'électricité et un deuxième organe conducteur (202, 212, 222) de l'électricité,
- Ledit élément de mesure (2) intégrant une première traversée électrique (203, 213, 223) reliant ladite électrode de mesure au premier organe conducteur et une deuxième traversée électrique (204, 214, 224) reliant ladite électrode de référence au deuxième organe conducteur,
- L'élément de mesure (2) comportant une tige (21) allongée suivant un axe dit longitudinal réalisée en matériau isolant de l'électricité, la tige (21) comprenant une extrémité proximale et une extrémité distale, ladite tige (21) portant à son extrémité proximale ledit premier organe conducteur (211, 221) de l'électricité et ledit deuxième organe conducteur (212, 222) de l'électricité, la tige (21) comprenant une surface externe destinée à venir en contact avec l'échantillon liquide (ECH), sur laquelle sont réalisées ladite électrode de mesure (E1) et ladite électrode de référence (E2),
- **Caractérisé en ce que** :
- La tige (21) comporte une première cavité réalisée sur sa surface externe, ladite électrode de référence étant déposée dans ladite première cavité,
- La tige (21) comporte une deuxième cavité réalisée sur sa surface externe, ladite électrode de mesure étant déposée dans ladite deuxième cavité.

2. Dispositif selon la revendication 1, **caractérisé en ce que** l'élément de mesure (2) comporte un bouchon (20) réalisé en matériau isolant de l'électricité et comprenant des moyens de coopération par vissage ou emboîtement, destinés à coopérer avec des moyens complémentaires agencés sur un goulot du conteneur (1).

3. Dispositif selon la revendication 1 ou 2, **caractérisé en ce que** la tige (21) comporte une pointe (217) à son extrémité distale.

4. Dispositif selon l'une des revendications 1 à 3, **caractérisé en ce que** la tige (21) est creuse sur toute sa longueur.

5. Dispositif selon l'une des revendications 1 à 4, **caractérisé en ce que** l'élément de mesure (2) comporte une tête (210, 22) positionnée à l'extrémité proximale de la tige (21), la tête portant ledit premier organe conducteur (211, 221) et ledit deuxième organe conducteur (212, 222).

6. Dispositif selon la revendication 5, **caractérisé en ce qu'**il comporte des moyens d'assemblage de la tête (210, 22) sur la tige (21).

7. Dispositif selon la revendication 6, **caractérisé en ce que** la tige (21) et la tête (210, 22) coopèrent entre elles par vissage.

8. Dispositif selon l'une des revendications 5 à 9, **caractérisé en ce que** la tête (22) comporte des moyens de coopération par vissage ou emboîtement, destinés à coopérer avec des moyens complémentaires agencés sur un goulot du conteneur.

9. Dispositif selon l'une des revendications 1 à 8, **caractérisé en ce que** la tige (21) est réalisée à partir d'une carte électronique réalisée en matériau souple et enroulée sur elle-même.

10. Dispositif selon l'une des revendications 1 à 9, **caractérisé en ce que** ladite électrode de mesure (E1) est réalisée sous la forme d'un dépôt d'une encre conductrice ou d'un électro-dépôt d'un élément de mesure conducteur.

11. Dispositif selon l'une des revendications 1 à 10, **caractérisé en ce que** ladite électrode de référence (E2) est réalisée sous forme d'un dépôt d'une encre Ag/AgCl recouverte d'une couche polymère.

## Patentansprüche

1. Instrumentierte Vorrichtung, die auf einen Behälter (1) aufsetzbar ist, der eine Hülle aufweist, die dazu bestimmt ist, eine flüssige Probe (ECH) aufzunehmen, wobei die Vorrichtung aufweist,
- Mindestens zwei Elektroden, die als Messelektrode (E1) und Referenzelektrode (E2) bezeichnet werden,
- Mindestens ein Messelement (2), das aus Elektrizität isolierendem Material ausgeführt ist,
- Wobei das Messelement (2) Mittel umfasst, um abnehmbar mit der Hülle des Behälters zusammenzuwirken,
- Wobei das Messelement (2) eine erste Oberfläche, die dazu bestimmt ist, mit einem Innenraum des Behälters in Kontakt zu gelangen, wenn das Messelement (2) auf den Behälter (1) aufgesetzt ist, und eine zweite entgegengesetzte, von außen zugängliche Oberfläche umfasst,
- Wobei die erste Oberfläche des Messelements (2) die Messelektrode (E1) und die Referenzelektrode (E2) trägt,
- Wobei die zweite Oberfläche des Messelements (2) ein erstes Elektrizität leitendes Organ (201, 211, 221) und ein zweites Elektrizität leitendes Organ (202, 212, 222) trägt,
- Wobei das Messelement (2) eine erste elektrische Durchführung (203, 213, 223), welche die Messelektrode mit dem ersten leitenden Organ verbindet, und eine zweite elektrische Durchführung (204, 214, 224), welche die Referenzelektrode mit dem zweiten leitenden Organ verbindet, beinhaltet,
- Wobei das Messelement (2) eine entlang einer Längsachse langgestreckte Stange (21) umfasst, die aus Elektrizität isolierendem Material ausgeführt ist, wobei die Stange (21) ein proximales Ende und ein distales Ende aufweist, wobei die Stange (21) an ihrem proximalen Ende das erste Elektrizität leitende Organ (211, 221) und das zweite Elektrizität leitende Organ (212, 222) trägt, wobei die Stange (21) eine Außenoberfläche aufweist, die dazu bestimmt ist, mit der flüssigen Probe (ECH) in Kontakt zu gelangen, und auf der die Messelektrode (E1) und die Referenzelektrode (E2) ausgeführt sind,
- **Dadurch gekennzeichnet, dass**:
- Die Stange (21) eine erste Kavität umfasst, die auf ihrer Außenoberfläche ausgeführt ist, wobei die Referenzelektrode in der ersten Kavität angeordnet ist,
- Die Stange (21) eine zweite Kavität umfasst, die auf ihrer Außenoberfläche ausgeführt ist, wobei die Messelektrode in der zweiten Kavität angeordnet ist.

2. Vorrichtung nach Anspruch 1, **dadurch gekennzeichnet, dass** das Messelement (2) einen Stopfen (20) umfasst, der aus Elektrizität isolierendem Material ausgeführt ist und Mittel zum Zusammenwirken durch Schrauben oder Stecken aufweist, die dazu bestimmt sind, mit komplementären Mitteln zusammenzuwirken, die an einem Hals des Behälters (1) ausgebildet sind.

3. Vorrichtung nach Anspruch 1 oder 2, **dadurch gekennzeichnet, dass** die Stange (21) an ihrem distalen Ende eine Spitze (217) umfasst.

4. Vorrichtung nach einem der Ansprüche 1 bis 3, **dadurch gekennzeichnet, dass** die Stange (21) über ihre gesamte Länge hohl ist.

5. Vorrichtung nach einem der Ansprüche 1 bis 4, **dadurch gekennzeichnet, dass** das Messelement (2) einen Kopf (210, 22) umfasst, der an dem proximalen Ende der Stange (21) positioniert ist, wobei der Kopf das erste leitende Organ (211, 221) und das zweite leitende Organ (212, 222) trägt.

6. Vorrichtung nach Anspruch 5, **dadurch gekennzeichnet, dass** sie Mittel zum Montieren des Kopfes (210, 22) an der Stange (21) umfasst.

7. Vorrichtung nach Anspruch 6, **dadurch gekennzeichnet, dass** die Stange (21) und der Kopf (210, 22) untereinander durch Schrauben zusammenwirken.

8. Vorrichtung nach einem der Ansprüche 5 bis 9, **dadurch gekennzeichnet, dass** der Kopf (22) Mittel zum Zusammenwirken durch Schrauben oder Stecken umfasst, die dazu bestimmt sind, mit komplementären Mitteln zusammenzuwirken, die an einem Hals des Behälters ausgebildet sind.

9. Vorrichtung nach einem der Ansprüche 1 bis 8, **dadurch gekennzeichnet, dass** die Stange (21) ausgehend von einer Elektronikplatine ausgeführt ist, die aus flexiblem Material ausgeführt und aufgerollt ist.

10. Vorrichtung nach einem der Ansprüche 1 bis 9, **dadurch gekennzeichnet, dass** die Messelektrode (E1) in Form einer Abscheidung einer leitenden Tinte oder einer Elektroabscheidung eines leitenden Messelements ausgeführt ist.

11. Vorrichtung nach einem der Ansprüche 1 bis 10, **dadurch gekennzeichnet, dass** die Referenzelektrode (E2) in Form einer Abscheidung einer Ag/AgCl-Tinte ausgeführt ist, die mit einer Polymerschicht überzogen ist.

## Claims

1. Instrumented device that can be fitted to a container (1) comprising a casing for receiving a liquid sample (ECH), the device comprising:
- At least two electrodes, referred to as the measuring electrode (E1) and reference electrode (E2),
- At least one measuring element (2) made of electrically insulating material,
- Said measuring element (2) including means for removably cooperating with the casing of said container,
- Said measuring element (2) including a first surface intended to come into contact with an internal volume of said container when said measuring element (2) is fitted on the container (1), and a second opposing surface accessible from the outside,
- The first surface of said measuring element (2) carrying said measuring electrode (E1) and said reference electrode (E2),
- The second surface of said measuring element (2) carrying a first electrically conductive member (201, 211, 221) and a second electrically conductive member (202, 212, 222),
- Said measuring element (2) incorporating a first electrical feedthrough (203, 213, 223) connecting said measuring electrode to the first conductive member and a second electrical feedthrough (204, 214, 224) connecting said reference electrode to the second conductive member,
- The measuring element (2) including a rod (21) elongated along a longitudinal axis made of electrically insulating material, the rod (21) comprising a proximal end and a distal end, said rod (21) carrying at its proximal end said first electrically conductive member (211, 221) and said second electrically conductive member (212, 222), the rod (21) comprising an outer surface intended to come into contact with the liquid sample (ECH), on which said measuring electrode (E1) and said reference electrode (E2) are formed,
- **Characterized in that**:
- The rod (21) includes a first cavity made on its outer surface, said reference electrode being deposited in said first cavity,
- The rod (21) includes a second cavity made on its outer surface, said measuring electrode being deposited in said second cavity.

2. Device according to Claim 1, **characterized in that** the measuring element (2) includes a plug (20) made of electrically insulating material and comprising means for cooperation by screwing or interlocking, intended to cooperate with complementary means arranged on a neck of the container (1).

3. Device according to Claim 1 or 2, **characterized in that** the rod (21) has a tip (217) at its distal end.

4. Device according to one of Claims 1 to 3, **characterized in that** the rod (21) is hollow over its entire length.

5. Device according to one of Claims 1 to 4, **characterized in that** the measuring element (2) includes a head (210, 22) positioned at the proximal end of the rod (21), the head carrying said first conductive member (211, 221) and said second conductive member (212, 222).

6. Device according to Claim 5, **characterized in that** it includes means for assembling the head (210, 22) on the rod (21).

7. Device according to Claim 6, **characterized in that** the rod (21) and the head (210, 22) cooperate with one another by screwing.

8. Device according to one of Claims 5 to 9, **characterized in that** the head (22) includes means for cooperation by screwing or interlocking, intended to cooperate with complementary means arranged on a neck of the container.

9. Device according to one of Claims 1 to 8, **characterized in that** the rod (21) is made from an electronic board made of flexible material and wound about itself.

10. Device according to one of Claims 1 to 9, **characterized in that** said measuring electrode (E1) is made in the form of a deposit of a conductive ink or an electrodeposit of a conductive measuring element.

11. Device according to one of Claims 1 to 10, **characterized in that** said reference electrode (E2) is produced in the form of a deposit of an Ag/AgCl ink covered with a polymer layer.
